# EUROPÄISCHE PATENTSCHRIFT

(11) **EP 0 712 841 B1**
(45) Veröffentlichungstag und Bekanntmachung des Hinweises auf die Patenterteilung: **13.01.1999**
(21) Anmeldenummer: 95117132.1
(22) Anmeldetag: 31.10.1995
(51) Int. Cl.: C07C 315/02, C07C 317/28, C07C 319/14, C07C 323/25, C07D 265/10

(54) **Verfahren zur Herstellung von 3-(N-Aryl-amino)-propyl-2'-sulfatoethyl-sulfonyl-Verbindungen**
Process for the preparation of 3(N-aryl-amino)-propyl-2'-sulfatoethylsulfonyl compounds
Procédé de préparation de composés de 3(N-arylamino)propyl-2'-sulfatoéthylsulfonyle

(30) Priorität: 18.11.1994 DE 4441147
(43) Veröffentlichungstag der Anmeldung: 22.05.1996
(73) Patentinhaber: DyStar Textilfarben GmbH & Co. Deutschland KG, 60318 Frankfurt am Main (DE)
(72) Erfinder: Schumacher, Christian, Dr., D-65779 Kelkheim (DE); Meier, Michael, Dr., D-60487 Frankfurt (DE); Russ, Werner Hubert, Dr., D-65349 Flörsheim (DE)

(56) Entgegenhaltungen:
- EP-A- 0 632 022
- WO-A-92/15559
- JOURNAL OF ORGANIC CHEMISTRY, Bd. 27, 1962, EASTON US, Seiten 3079 - 3083 R. J. STOFFEL ET AL 'The preparation of 2-imidazolones. A novel ring closure of propynylureas with phosphorous pentachloride'
- JOURNAL OF THE AMERICAN CHEMICAL SOCIETY, Bd. 45, 1923 DC US, Seiten 790-795, J. S. PIERCE ET AL 'Tetrahydro-1,3,2-oxazones and substituted gamma-amino propanols'

## Beschreibung

Die vorliegende Erfindung liegt auf dem technischen Gebiet der Reaktivfarbstoff-Vorprodukte.

3-(N-Aryl-amino)-propyl-2'-sulfatoethyl-sulfonyl-Verbindungen sind wertvolle Zwischenprodukte für die Herstellung von Reaktivfarbstoffen.

Der vorliegenden Erfindung lag die Aufgabe zugrunde, ein Verfahren zur Herstellung besagter Zwischenprodukte mit hoher Ausbeute und Reinheit zur Verfügung zu stellen.

Es wurde gefunden, daß die Aufgabe überraschenderweise gelöst wird, wenn man nachstehend beschriebene Verfahrensschritte durchführt.

Gegenstand der Erfindung ist ein Verfahren zur Herstellung von Verbindungen der Formel (1)

Ar-NH-(CH₂)₃-SO₂-(CH₂)₂-OSO₃M (1)

worin
- M: für Alkalimetall oder für Wasserstoff steht,
- Ar: für einen aromatischen Rest der Formel 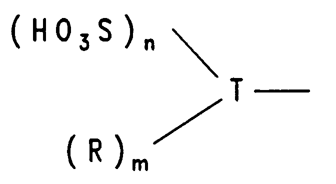
steht, worin
- n: für eine Zahl von 0 bis 3 steht,
- m: für eine Zahl von 0 bis 3 steht,
- R: für C₁-C₄-Alkyl, C₁-C₄-Alkoxy, Amino, C₁-C₄-Alkylamino, Di-(C₁-C₄)-alkylamino, C₆-C₁₀-Arylamino, C₁-C₄-Acylamino, Ureido, Nitro oder Cyano steht, wobei C₁-C₄-Alkyl und C₆-C₁₀-Aryl durch ein bis drei Reste aus der Reihe C₁-C₄-Alkoxy, Halogen, Hydroxy, Carboxy, Sulfo, Sulfato, Nitro, Cyano oder eine Kombination davon substituiert sein können,
und
- T: für ein aromatisches Brückenglied aus der Benzol- oder Naphthalinreihe
steht,
dadurch gekennzeichnet, daß man eine Verbindung der Formel (2) 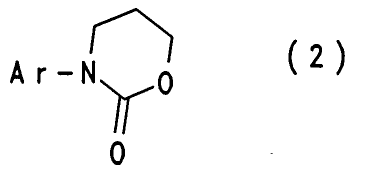 mit 2-Mercaptoethanol zu einer Verbindung der Formel (3)

Ar-NH-(CH₂)₃-S-(CH₂)₂-OH (3)

bei Temperaturen von 90 bis 270°C in Gegenwart einer katalytischen Menge einer Base umsetzt,
die Verbindung der Formel (3) zu einer Verbindung der Formel (4) oxidiert

Ar-NH-(CH₂)₃-SO₂-(CH₂)₂-OH (4),

und die Verbindung der Formel (4) mit mindestens einem Mol-Equivalent Schwefelsäure, Oleum oder Halogensulfonsäure zu einer Verbindung der Formel (1) verestert.

Bevorzugt im Sinne der vorliegenden Erfindung sind Verbindungen der Formel (1), worin
- M: Wasserstoff, Natrium oder Kalium ist,
- n: für die Zahl 0 oder 1,
- m: für die Zahl 0 oder 1, vorzugsweise 0,
- R: für Methyl, Ethyl, Methoxy, Ethoxy, Methylamino, insbesondere für Amino und
- A: r für Phenyl, 2-Sulfophenyl, 3-Sulfophenyl, 4-Sulfophenyl, 2,5-Disulfophenyl, 2-Methylphenyl, 3-Methylphenyl, 4-Methylphenyl, 2-Methoxyphenyl, 3-Methoxyphenyl, 4-Methoxyphenyl, 3-Aminophenyl, 4-Aminophenyl, 3-Sulfo-4-aminophenyl, 4-Sulfo-3-aminophenyl, 3-Methoxy-5-sulfophenyl, 3-Sulfo-4-methylphenyl, 3-Sulfo-4-methoxyphenyl, 2-Sulfo-4-methylphenyl, 4-Methyl-2-sulfophenyl, 2-Sulfo-4-methoxyphenyl, 2,5-Disulfo-4-methylphenyl, 2,5-Disulfo-4-methoxyphenyl, 2,5-Disulfo-4-aminophenyl, 5,7-Disulfo-naphth-2-yl, 4,8-Disulfo-naphth-2-yl, 3,6,8-Trisulfo-naphth-2-yl, 4,6,8-Trisulfo-naphth-2-yl, 8-Sulfo-naphth-1-yl, 6-Sulfo-naphth-1-yl oder 7-Sulfo-naphth-1-yl stehen.

Beispiele für besonders bevorzugte 3-(N-Aryl-amino)-propyl-2'-sulfatoethyl-sulfonyl-Verbindungen der Formel (1) sind die nachfolgenden Verbindungen (1a), (1b) und (1c) 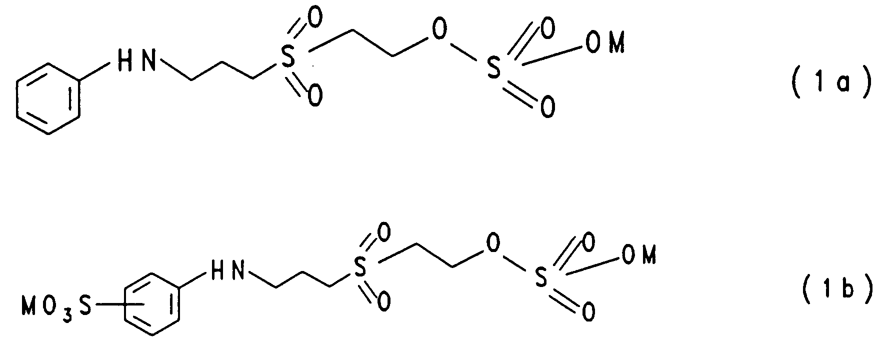 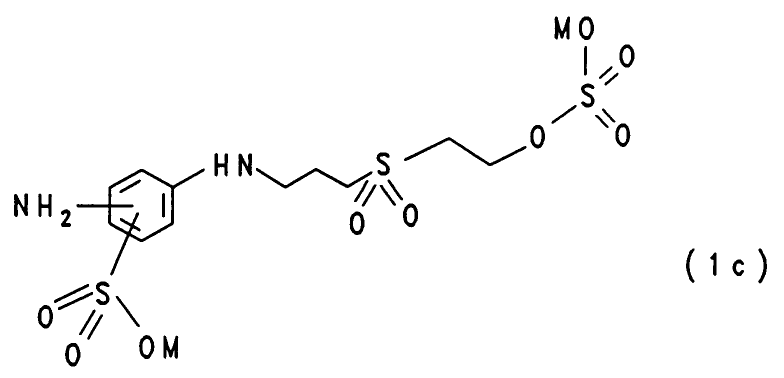 worin M Wasserstoff oder Alkalimetall, vorzugsweise Na, bedeutet.

Das erfindungsgemäße Verfahren geht von einem 3-Aryl-tetrahydro-2H-1,3-oxazin-2-on der Formel (2) aus, welches mit 1 bis 3-fach molaren Mengen an Mercaptoethanol in Gegenwart einer katalytischen Menge Base, wie eines Alkali- oder Erdalkalihydroxids, -carbonats, -phosphats, -alkoholats, bevorzugt Natriumcarbonat, Kaliumcarbonat, Cäsiumcarbonat, Natriumhydroxid, Kaliumhydroxid oder Natriummethanolat, in Substanz oder in einem der nachstehend genannten Lösemittel oder in einem Lösemittel-Wasser-Gemisch, bevorzugt in Substanz oder in einem aliphatischen Kohlenwasserstoff, bei einer Temperatur von 90 bis 270°C, bevorzugt 160 bis 240°C, bevorzugt in einer Inertgasatmosphäre und gegebenenfalls unter reduziertem Druck, unter Ringöffnung und CO₂-Abspaltung zu neuen 3-(N-Arylamino)-propyl-2'-hydroxyethyl-sulfid-Verbindungen der Formel (3) umgesetzt wird.

Unter einer katalytischen Menge werden Konzentrationen von 1 bis 15 Mol-%, bezogen auf die Verbindung der Formel (2), verstanden.

Geeignete Lösemittel sind beispielsweise Alkohole, Ether oder Amide, wie Ethanol, Propanol, Isopropanol, Dimethoxyethan, Diethylenglykoldimethylether, Dimethylacetamid, N-Methylpyrrolidon und/oder aliphatische Kohlenwasserstoffe wie Decan oder Undecan.

Ringöffnungen von cyclischen Urethanen unter Abspaltung von CO₂ sind bereits prinzipiell bekannt, z.B. die Ringöffnung von cyclischen 5-Ring-Urethanen durch Alkoholat zu 2-Alkoxyethyl-anilin-Derivaten in Tetrahedron Letters 29 (1988), 5095 oder die Hydrolyse von 3-Phenyl-tetrahydro-2H-1,3-oxazin-2-on zu 3-Hydroxypropyl-anilin in C.R. Hebd. Seances Acad. Sci. Ser. C. (1975), 280(20), 1269. Aus J. Am. Chem. Soc. 45 (1923), 723 sind solche Verbindungen der Formel (2) bekannt, in denen Ar die Bedeutung Phenyl oder o-Methylphenyl hat.

Verbindungen der Formel (2a) 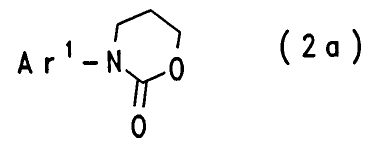 worin
- Ar¹: 2-Sulfophenyl, 3-Sulfophenyl, 4-Sulfophenyl, 2,5-Disulfophenyl, 3-Sulfo-4-aminophenyl, 4-Sulfo-3-aminophenyl, 3-Methoxy-5-sulfophenyl, 3-Sulfo-4-methylphenyl, 3-Sulfo-4-methoxyphenyl, 2-Sulfo-4-methylphenyl, 2,5-Disulfo-4-methylphenyl, 2,5-Disulfo-4-methoxyphenyl, 2,5-Disulfo-4-aminophenyl, 5,7-Disulfo-naphth-2-yl, 4,8-Disulfo-naphth-2-yl, 3,6,8-Trisulfo-naphth-2-yl, 4,6,8-Trisulfo-naphth-2-yl, 8-Sulfo-naphth-1-yl, 6-Sulfo-naphth-1-yl oder 7-Sulfo-naphth-1-yl und
- M: Alkalimetall oder Wasserstoff bedeutet,
sind neu und Gegenstand der vorliegenden Erfindung.

Das nachfolgend beschriebene Verfahren zur Herstellung von cyclischen Urethanen der Formel (2) und (2a) hat im Vergleich zu dem in J. Am. Chem. Soc. 45 (1923), 723 beschriebenen Verfahren Vorteile im Hinblick auf Ausbeute und Wirtschaftlichkeit.

Cyclische Urethane der Formel (2) werden hergestellt, indem man eine offenkettige Verbindung der Formel (8) 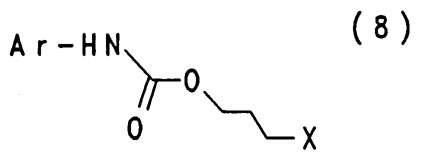 worin
- Ar: die vorstehend definierte Bedeutung hat,
- X: für eine Abgangsgruppe aus der Reihe Halogen, wie Chlor, Brom oder Iod, Sulfato, C₁-C₄-Alkoxy, wie Methoxy oder Ethoxy, C₁-C₄-Alkylcarbonyloxy, wie Acetyloxy, (C₆-C₁₀-Aryl)carbonyloxy, C₁-C₄-Alkylsulfonyloxy, (C₆-C₁₀-Aryl)sulfonyloxy oder einen Rest NR¹R²R³, worin die Reste R¹, R², R³ für C₁-C₄-Alkyl, wie Methyl oder Ethyl, C₆-C₁₀-Aryl-C₁-C₄-alkylen, wie Benzyl, oder C₆-C₁₀-Aryl, wie Phenyl, stehen, wobei C₁-C₄-Alkyl oder C₆-C₁₀-Aryl-C₁-C₄-alkylen bevorzugt sind, mit der Maßgabe, daß mindestens zwei der Reste R¹, R² und R³ für C₁-C₄-Alkyl stehen, bevorzugt steht X dabei für die Reste Chlor,Brom, Sulfato, Methoxy, Ethoxy, Acetyloxy, Dimethylbenzylammonium, Trimethylammonium und insbesondere bevorzugt für Chlor;
unter Eliminierung von HX durch die Einwirkung von 0,8 bis 1,2 Moläquivalenten einer Base bei einem pH-Wert von 10 bis 13, bevorzugt 11 bis 12, bei Temperaturen von 25 bis 100°C, bevorzugt 50 bis 80°C, zu einem cyclischen Urethan der Formel (2) umsetzt. In gleicher Weise werden cyclische Urethane der Formel (2a) hergestellt.

Die Umsetzung kann in Wasser, einem der vorstehend genannten Lösemittel oder in einem Lösemittel-Wasser-Gemisch erfolgen.

Beispiele für geeignete Basen sind Alkali- oder Erdalkalihydroxide, -phosphate, -silicate, -alkoholate oder -carbonate, wobei Alkali-alkoholate und -hydroxide bevorzugt sind. Die bevorzugte erfindungsgemäße Verfahrensvariante ist weiterhin dadurch gekennzeichnet, daß die Base langsam zudosiert wird, d.h. während einer Zeit von 1 bis 4 Stunden. Für den Fall, daß der Rest Ar keine Sulfogruppe trägt (n = 0), wird die Umsetzung bevorzugt in einem Alkohol, wie Isopropanol oder Ethanol, durchgeführt. Für den Fall, daß Ar 1 bis 3 Sulfogruppen trägt (n = 1 bis 3), wird die Umsetzung bevorzugt in Wasser durchgeführt.

Die so hergestellten Verbindungen der Formel (2) können durch Kristallisation bei Temperaturen von unter 20°C gereinigt werden, z.B. aus Ethanol bei einer Temperatur von -20 bis 5°C für den Fall, daß Ar für Phenyl oder für Sulfophenyl steht.

Verbindungen der Formel (8) können aus einem Amin der Formel

Ar-NH₂

und einer Verbindung der Formel (9)

Cl-COO-CH₂-CH₂-CH₂-X (9),

worin Ar und X eine der vorstehend genannten Bedeutungen haben, in Wasser oder in einem Lösemittel in Gegenwart einer Base hergestellt werden. Die Verbindung der Formel (9) ist beispielsweise Chlorameisensäure-3-chlorpropylester. Diese Verfahrensweise ist in J. Am. Chem. Soc. 45, 723 (1923) bereits prinzipiell beschrieben. Das erfindungsgemäße Verfahren ist dadurch gekennzeichnet, daß das Amin in Wasser, in einem Lösemittel oder in einem Lösemittel-Wasser-Gemisch vorgelegt wird und die Verbindung der Formel (9) und die Base gleichzeitig unter pH-Kontrolle langsam zudosiert werden, wodurch bessere Ausbeuten und eine höhere Reinheit als bei der erwähnten bekannten Verfahrensweise erreicht werden. Das erfindungsgemäße Verfahren ist für den Fall, daß Ar 1 bis 3 Sulfogruppen trägt, dadurch gekennzeichnet, daß man die Umsetzung in Wasser bei 10 bis 30°C bei einem pH-Wert von 6 bis 8 durchführt und für den Fall, daß Ar frei von Sulfogruppen ist, dadurch gekennzeichnet, daß man die Umsetzung in einem Lösemittel oder in einem Wasser-Lösemittel-Gemisch bei 10 bis 30°C und bei einem pH-Wert von 6 bis 8 durchführt. Als Basen und Lösemittel kommen die bereits vorstehend beschriebenen in Betracht.

Eine alternative Verfahrensvariante zur Herstellung einer Verbindung der Formel (8) ist dadurch gekennzeichnet, daß man ein Isocyanat der Formel

Ar-N=C=O

und eine Verbindung der Formel (10)

HO-CH₂-CH₂-CH₂-X (10),

worin Ar und X eine der vorstehend genannten Bedeutungen haben, zu einer Verbindung der Formel (8) miteinander umsetzt. Die Verbindung der Formel (10) ist beispielsweise 3-Chlorpropanol, 3-Brompropanol, 3-(Alkylcarbonyloxy)propanol oder 3-Sulfato-propanol. Diese Verfahrensweise ist in J. Am. Chem. Soc. 45, 723 (1923) bereits prinzipiell beschrieben. Dort wird die Umsetzung bei 150°C durchgeführt (X=Cl), was für die Ausbeuten und Produktqualitäten nachteilig ist. Besser verläuft die Umsetzung im allgemeinen bei Temperaturen von 10 bis 120°C, bevorzugt 20 bis 90°C, und für den Fall, daß X Chlor ist, bevorzugt bei 55 bis 80°C, in Substanz oder in einem in Bezug auf die Reaktionsbedingungen inerten Lösemittel, wie z.B. Toluol.

Das Verfahren unter Verwendung des Isocyanats der Formel Ar-N=C=O wird bei sulfogruppenfreien Resten Ar bevorzugt .

Das Verfahren unter Verwendung des Amins der Formel Ar-NH₂ wird bei Resten Ar, die 1 bis 3 Sulfogruppen tragen, bevorzugt .

Eine weitere erfindungsgemäße Verfahrensvariante für die Herstellung von Verbindungen der Formel (2), vorzugsweise für Verbindungen, worin Ar frei von Sulfogruppen ist, ist dadurch gekennzeichnet, daß man eine N-Aryl-3-hydroxypropylamin-Verbindung der Formel (11)

Ar-NH-(CH₂)₃-OH (11),

deren Herstellung beispielsweise in J. Org. Chem. 58 (1993), 6235 für Ar gleich Phenyl beschrieben ist, mit einer Verbindung der Formel

X¹-CO-X² (12)

worin
- X¹: für Halogen, bevorzugt Chlor, C₁-C₄-Alkoxy, bevorzugt Methoxy oder Ethoxy, steht, und
- X²: unabhängig von X¹ eine der Bedeutungen von X¹ hat, umsetzt.

Beispiele für bevorzugte Verbindungen der Formel (12) sind Phosgen, Chlorkohlensäuremethylester, Chlorkohlensäureethylester, Dimethylcarbonat oder Diethylcarbonat. Erfindungsgemäß werden die Verbindungen der Formel (2) aus den genannten Verbindungen der Formel (11) und (12) bei Temperaturen von 80 bis 140°C für den Fall, daß eine oder beide der Reste X¹ oder X² für C₁-C₄-Alkoxy stehen, oder bei 20 bis 140°C für den Fall, daß X¹ und X² beide Halogen sind, in Gegenwart einer Base hergestellt.

Als Base kommt für den Fall daß X¹ und X² für C₁-C₄-Alkoxy stehen, eine katalytische Menge Base oder für den Fall, daß einer der Reste X¹ oder X² für Halogen steht, eine molare Menge Base oder für den Fall, daß beide Reste X¹ und X² für Halogen stehen, eine zweifach molare Menge Base in Betracht. Als Base sind besonders Alkalialkoholate, beispielsweise Natriummethanolat, von Interesse.

Die durch die Umsetzung eines cyclischen Urethans der Formel (2) mit 2-Mercaptoethanol hergestellten Zwischenverbindungen der Formel (3) sind bisher noch nicht bekannt und Gegenstand der vorliegenden Erfindung. Bevorzugt sind Verbindungen der Formel (3), worin Ar die Bedeutung Phenyl, 2-Sulfophenyl, 3-Sulfophenyl, 4-Sulfophenyl, 2,5-Disulfophenyl, 2-Methylphenyl, 3-Methylphenyl, 4-Methylphenyl, 2-Methoxyphenyl, 3-Methoxyphenyl, 4-Methoxyphenyl, 3-Aminophenyl, 4-Aminophenyl, 3-Sulfo-4-aminophenyl, 4-Sulfo-3-aminophenyl, 3-Methoxy-5-sulfophenyl, 3-Sulfo-4-methylphenyl, 3-Sulfo-4-methoxyphenyl, 2-Sulfo-4-methylphenyl, 4-Methyl-2-sulfophenyl, 2-Sulfo-4-methoxyphenyl, 2,5-Disulfo-4-methylphenyl, 3,5-Disulfo-4-methoxyphenyl, 2,5-Disulfo-4-aminophenyl, 5,7-Disulfo-naphth-2-yl, 4,8-Disulfo-naphth-2-yl, 3,6,8-Trisulfo-naphth-2-yl, 4,6,8-Trisulfo-naphth-2-yl, 8-Sulfo-naphth-1-yl, 6-Sulfo-naphth-1-yl oder 7-Sulfo-naphth-1-yl hat und M Wasserstoff oder Alkalimetall bedeutet.

Gegenstand der Erfindung ist auch ein Verfahren zur Herstellung einer Verbindung der Formel (1), dadurch gekennzeichnet, daß man von einer Verbindung der Formel (3) ausgeht und diese bei Temperaturen von 90 bis 270°C in Gegenwart einer katalytischen Menge einer Base umsetzt, die Verbindung der Formel (3) zu einer Verbindung der Formel (4) oxidiert

Ar-NH-(CH₂)₃-SO₂-(CH₂)₂-OH (4),

und die Verbindung der Formel (4) mit mindestens einem Mol-Equivalent Schwefelsäure, Oleum oder Halogensulfonsäure zu einer Verbindung der Formel (1) verestert.

Die Verbindung der Formel (3) wird in Substanz oder zweckmäßigerweise in einem in bezug auf die Reaktionsbedingungen inerten organischen Lösemittel, beispielsweise N-Methylpyrrolidon oder Dimethylacetamid, zur Verbindung der Formel (4) oxidiert, vorzugsweise mit Wasserstoffperoxid als Oxidationsmittel und in Gegenwart einer vorzugsweise katalytischen Menge einer Übergangsmetall-Sauerstoff-Verbindung, wie Natriumwolframat, Wolframoxid oder Natriumvanadat, bei einer Temperatur zwischen 70 und 110°C. Unter katalytischer Menge werden hier 0,2 bis 5, bevorzugt 0,5 bis 2, Gew.-Teile der genannten Übergangsmetallverbindung pro Mol Thioether der Formel (3) verstanden. Die Menge an Wasserstoffperoxid beträgt zweckmäßigerweise 1,8 bis 2,2 Mol-Äquivalente, bezogen auf die zu oxidierende Verbindung der Formel (3).

Die Verbindungen der Formel (4) werden mit 1 bis 1,5 Molequivalenten, vorzugsweise 1,05 bis 1,2 Molequivalenten, Schwefelsäure, enthaltend gegebenenfalls Anteile an SO₃, oder mit 1 bis 1,5, vorzugsweise 1,05 bis 1,2, Molequivalenten einer Halogensulfonsäure, vorzugsweise Chlorsulfonsäure, zu Sulfatoethylsulfon-Verbindungen der Formel (1) verestert (M = Wasserstoff).

Es ist zweckmäßig, vor der Veresterung das Reaktionsgemisch durch Einwirkung von Wärme und/oder reduziertem Druck von Wasser zu befreien und die Veresterung ohne Zwischenisolierung des Sulfons der Formel (4) bei Temperaturen zwischen 0 und 40°C, bevorzugt 10 und 30°C, in dem besagten inerten organischen Lösemittel durchzuführen.

Anschließend wird die Verbindung der Formel (1) als Sulfosäure (M = H) oder als Alkalimetallsalz (M = Li, Na oder K) isoliert, wobei die Isolierung als Alkalimetallsalz, insbesondere als Natriumsalz, bevorzugt ist. Hierzu wird das besagte Lösemittel durch Destillation, bevorzugt unter reduziertem Druck, entfernt.

In einer alternativen Ausführungsform wird die 3-(N-Arylamino)-propyl-2'-hydroxyethyl-sulfid-Verbindung der Formel (3) in Wasser mit einem Anhydrid oder Säurechlorid, beispielsweise mit Acetylchlorid, Trifluoracetylchlorid, Acetanhydrid oder Trifluoracetanhydrid, zu einer 3-(N-Aryl-acyl-amino)-propyl-2'-hydroxyethyl-sulfid-Verbindung der Formel (7) umgesetzt

Ar-N(acyl)-(CH₂)₃-S-(CH₂)₂-OH (7)

worin acyl für einen gegebenenfalls substituierten C₁-C₄-Alkylcarbonyl-Rest, bevorzugt Acetyl, Trichloracetyl oder Trifluoracetyl, steht.
Die Oxidation der Verbindung der Formel (7) zur Sulfonylverbindung erfolgt wie bereits beschrieben, ist jedoch dadurch gekennzeichnet, daß sie außer in Substanz oder in den genannten organischen Lösemitteln auch in Wasser durchgeführt werden kann, wobei Wasser bevorzugtes Lösemittel ist.

Aus der so erhaltenen Sulfonylverbindung wird die Acylgruppe im alkalischen oder sauren Bereich, vorzugsweise in saurer, salzsaurer oder schwefelsaurer, wäßriger Lösung, wie beispielsweise in 5 bis 30 gew.-%iger wäßriger Salzsäure oder Schwefelsäure für den Fall, daß Acyl für Acetyl steht, bei einer Temperatur zwischen 80 und 100°C hydrolytisch abgespalten, wobei die Verbindung der Formel (4) in wäßriger Lösung erhalten wird.

Die durch das erfindungsgemäße Verfahren hergestellten Verbindungen der Formel (1) zeichnen sich durch eine Ausbeute von 70 bis 80 % (nur organische Anteile, das isolierte Produkt enthält noch anorganische Salze) und eine Reinheit von 80 bis 90 %, über alle Synthesestufen gerechnet, aus und werden insbesondere zur Herstellung von Reaktivfarbstoffen zum Färben von Fasermaterialien verwendet (EP-A-0 629 667).

In den nachfolgenden Beispielen bedeuten "Teile" Gewichtsteile.

### Beispiel 1 Ar = Phenyl

### Stufe 1

119 Teile Phenylisocyanat werden zu 120 Teilen vorgelegtes 3-Chlorpropanol bei 60°C innerhalb von 2 Stunden zudosiert. Anschließend wird bei dieser Temperatur unter Rühren noch einige Zeit weiter umgesetzt und man erhält Chlorpropylphenylurethan der Formel 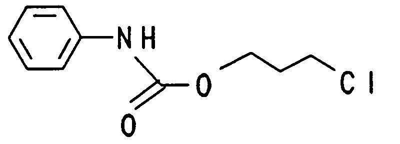 als Öl, das beim Kühlen im Eisbad kristallisiert. Die Umsetzung ist nahezu quantitativ.

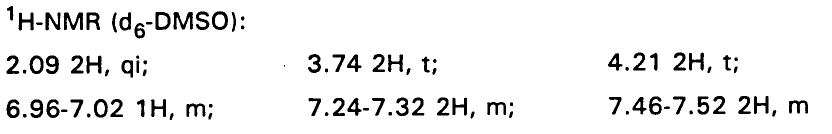

### Stufe 2

### a) Variante 1

Zu dem Öl aus Stufe 1 werden 500 ml Isopropanol gegeben. Innerhalb von 2 Stunden werden sodann 120 Teile einer 33 gew.-%igen Natronlauge bei einer Temperatur von 70°C zudosiert. Man entfernt anschließend das Lösungsmittel unter reduziertem Druck und kristallisiert das Produkt aus Ethanol bei einer Temperatur von -5 bis + 5°C. Die Kristalle werden unter reduziertem Druck bei 60°C getrocknet und man erhält 150 Teile N-Phenyltetrahydro-oxazinon-2 der Formel

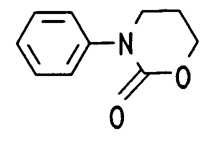

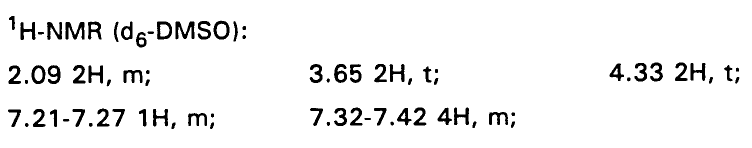

### b) Variante 2

213,7 Teile Chlorpropylphenylurethan aus Stufe 1 werden mit 1000 Teilen 2-Propanol vorgelegt und 50 Teile Natriumhydroxid in 2 Stunden bei 50°C zudosiert. Anschließend wird auf 85°C erhitzt und 4 Stunden bei dieser Temperatur nachgerührt. Die Suspension wird filtriert und der Filterkuchen mit 200 Teilen Isopropanol gewaschen. Das Filtrat wird zur Trockene eingedampft. Man erhält 173,9 Teile N-Phenyltetrahydro-oxazinon-2 mit einem Reingehalt von 88 %. Dies entspricht einer Ausbeute von 86 % d.Th.

### c) Variante 3

428 Teile Chlorpropylphenylurethan aus Stufe 1 werden mit 428 Teilen Dimethoxyethan vorgelegt und 85 Teile Natriumhydroxid in 4 Stunden bei 50°C zudosiert und innerhalb von 3 Stunden auf 80°C erhitzt. Anschließend wird 4 Stunden bei dieser Temperatur nachgerührt. Die Suspension wird bei 70°C filtriert und der Filterkuchen mit 214 Teilen Dimethoxyethan gewaschen. Das Filtrat wird zur Trockene eingedampft. Der Rückstand wird bei 0°C mit 214 Teilen Dimethoxyethan behandelt. Man erhält 306,6 Teile N-Phenyltetrahydrooxazinon-2 mit einem Reingehalt von > 99 %. Dies entspricht einer Ausbeute von 86 % d.Th. Aus der Mutterlauge können weitere 20 Teile Produkt erhalten werden.

### Stufe 3

### a) Variante 1

95 Teile 2-Mercaptoethanol werden unter Stickstoffatmosphäre mit 12 Teilen einer 33 gew.-%igen Natronlauge versetzt. Sodann werden 177 Teile der Verbindung aus Stufe 2 als Feststoff eingestreut. Man führt die Umsetzung bei 150°C unter Stickstoffatmosphäre zu Ende. Flüchtige Substanzen im Reaktionsgemisch werden sodann unter reduziertem Druck abdestilliert. Man erhält 210 Teile 3-(N-Phenylamino)-propyl-2'-hydroxyethylsulfid der Formel 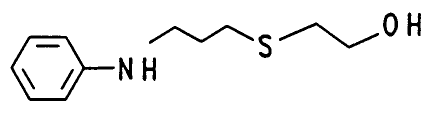 mit einer Reinheit von 80 % als gelbes Öl; dies entspricht einer Ausbeute von 80 % d.Th.

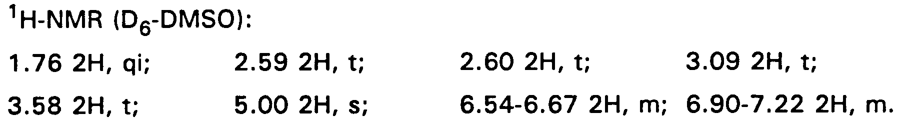

### b) Variante 2

177 Teile N-Phenyltetrahydro-oxazinon-2 werden mit 0,5 Teilen Kaliumcarbonat und 86 Teilen 2-Mercaptoethanol versetzt und unter Stickstoffatmosphäre auf 225°C erhitzt. Bei dieser Temperatur wird 1,5 Stunden nachgerührt. Man erhält 215 Teile 3-(N-Phenylamino)-propyl-2'-hydroxyethylsulfid mit einer Reinheit von 84 %; dies entspricht einer Ausbeute von 86 %.

### c) Variante 3

177 Teile N-Phenyltetrahydro-oxazinon-2 werden mit 1,0 Teilen Cäsiumcarbonat und 82 Teilen 2-Mercaptoethanol versetzt und unter Stickstoffatmosphäre auf 190°C erhitzt. Bei dieser Temperatur wird 6 Stunden nachgerührt. Man erhält 208 Teile 3-(N-Phenylamino)-propyl-2'-hydroxyethylsulfid mit einer Reinheit von 85 %; dies entspricht einer Ausbeute von 83 %.

### d) Variante 4

177 Teile N-Phenyltetrahydro-oxazinon-2 werden mit 0,5 Teilen Kaliumcarbonat, 86 Teilen 2-Mercaptoethanol und 40 Teilen Decan versetzt und unter Stickstoffatmosphäre auf 169°C erhitzt. Bei dieser Temperatur wird 6 Stunden gerührt. Anschließend wird das Lösemittel im Vakuum abdestilliert. Man erhält 216 Teile 3-(N-Phenylamino)-propyl-2'-hydroxyethylsulfid mit einer Reinheit von 87 %; dies entspricht einer Ausbeute von 89 %.

### Stufe 4

210 Teile 3-(N-Phenylamino)-propyl-2'-hydroxyethylsulfid werden bei 25°C mit 1000 Teilen wasserfreiem Dimethylacetamid und 1 Teil Natriumwolframat versetzt. Sodann gibt man bei einer Temperatur von 80 bis 90°C innerhalb von 4 Stunden 194 Teile einer 35 gew.-%igen wäßrigen Lösung von Wasserstoffperoxid hinzu. Es wird noch 1 Stunde bei dieser Temperatur nachgerührt, und man erhält 242 Teile der Verbindung der Formel 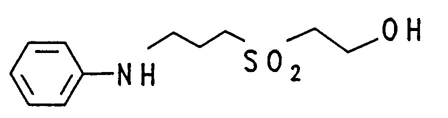 als Lösung in Dimethylacetamid. Die Umsetzung ist nahezu quantitativ.

### Stufe 5

### Variante 1:

Die Dimethylacetamid enthaltende Lösung aus Stufe 4 wird sodann im Vakuum von Wasser weitgehend befreit. Der verbleibende Sumpf wird bei 10 bis 25°C unter Eiskühlung langsam mit 125 Teilen Chlorsulfonsäure versetzt. Man rührt noch einige Zeit nach, neutralisiert durch Einstreuen von festem Natriumcarbonat bis pH 4 bis 5, filtriert von anorganischen Bestandteilen ab, destilliert die flüchtigen Bestandteile unter reduziertem Druck bei einer Sumpftemperatur von bis 60°C weitgehend ab und erhält 335 Teile der Verbindung der Formel 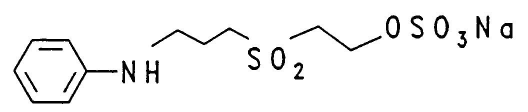 als ca. 40 %ige Lösung in Dimethylacetamid, die für weitere Synthesen, beispielsweise zur Herstellung von Farbstoffen ohne weitere Isolierung verwendet wird.

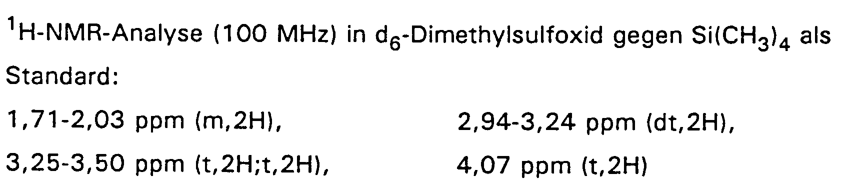

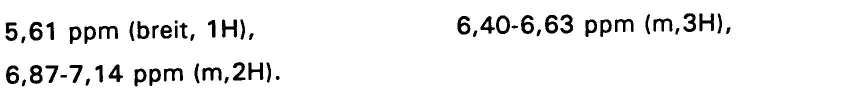

### Variante 2:

Das Verfahren gemäß Stufe 4 wird mit N-Methylpyrrolidon anstelle von Dimethylacetamid als Lösemittel und unter Verwendung von 119 Teilen Chlorsulfonsäure durchgeführt. Man erhält 335 Teile der in Variante 1 genannten Verbindung.

### Beispiel 2 Ar = 3-Sulfophenyl

### Stufe 1 und 2 kombiniert:

173 Teile 3-Sulfoanilin werden in 500 Teilen Wasser bei einem pH-Wert von 7 gelöst. Man tropft innerhalb von 2 Stunden 165 Teile Chlorameisensäure-3-chlorpropylester bei 20 bis 25°C zu, wobei der pH-Wert mit 10%iger Sodalösung bei 6 bis 7 gehalten wird. Man rührt noch einige Zeit nach und erhöht sodann den pH-Wert auf 11,5 bis 12. Bei einer Temperatur von 70°C wird bei diesem pH-Wert die Umsetzung zu Ende geführt. Der Ansatz wird im Vakuum eingedampft, aus Ethanol kristallisiert und man erhält die Verbindung der Formel

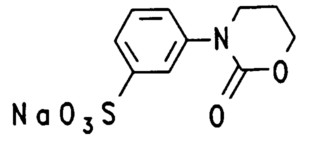

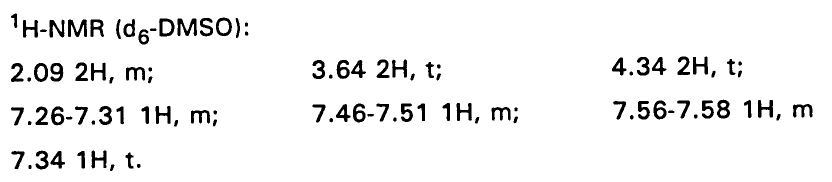

### Stufe 3

90 Teile Mercaptoethanol werden in einer Inertgasatmosphäre mit 8 Teilen Natriummethylat versetzt. Man erhitzt auf 80°C, treibt im Gasstrom Methanol aus und streut sodann 313 Teile der Verbindung aus der vorangegangenen Stufe 1 und 2 ein. Man erhitzt sodann auf 140 bis 150°C. Es wird noch eine Weile nachgerührt, bis die Umsetzung beendet ist. Man isoliert eine Verbindung der Formel 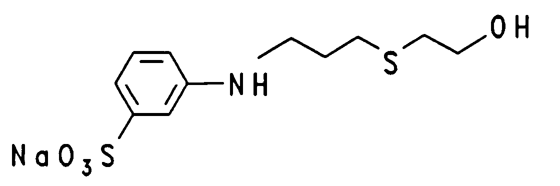 durch Einrühren des erhaltenen Öls in Ethanol, wobei das Produkt als weißer Feststoff anfällt. Es wird filtriert, mit Ethanol gewaschen und im Vakuum getrocknet.

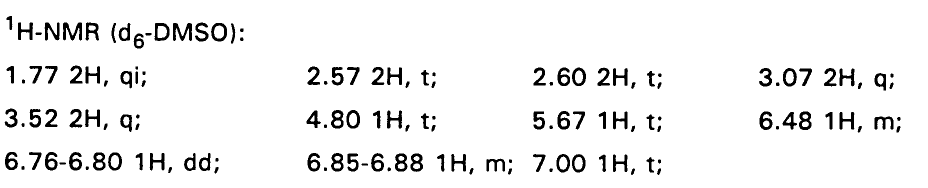

### Stufe 4 und 5 kombiniert:

238 Teile der Verbindung aus Stufe 3 werden in 1000 Teile Dimethylacetamid eingetragen, unter Rühren gelöst und mit 1 Teil NaVO₃ versetzt. Man gibt dann bei einer Temperatur von 80 bis 90°C 130 Teile Wasserstoffperoxid als ca. 40 gew.-%ige Lösung in Dimethylacetamid hinzu und rührt nach, bis die Umsetzung beendet ist. Man destilliert vorhandenes Wasser im Vakuum ab. Sodann werden langsam bei einer Temperatur von 10 bis 20°C 116 Teile Chlorsulfonsäure zugetropft. Man isoliert wie in Beispiel 1 angegeben und erhält die Verbindung der Formel

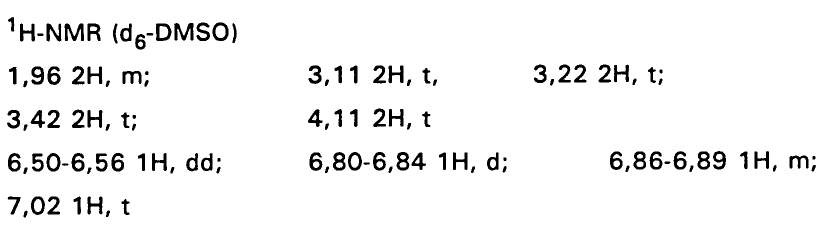

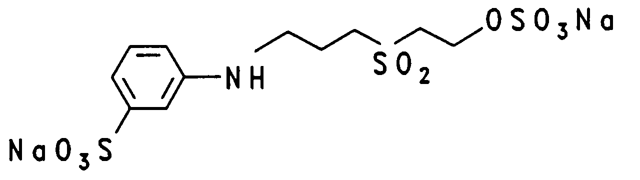

### Beispiel 3 Ar = 4-Sulfo-phenyl

### Stufe 1 und 2 kombiniert:

173 Teile 4-Sulfoanilin werden in 500 Teilen Wasser bei einem pH-Wert von 7 gelöst. Man tropft innerhalb von 2 Stunden 165 Teile Chlorameisensäure-3-chlorpropylester bei 20 bis 25°C zu, wobei der pH-Wert mit 10 %iger Sodalösung bei 6 bis 7 gehalten wird. Man rührt noch einige Zeit nach und erhöht sodann den pH-Wert auf 11,5 bis 12. Bei einer Temperatur von 70°C wird bei diesem pH-Wert die Umsetzung zu Ende geführt. Der Ansatz wird im Vakuum eingedampft und man erhält die Verbindung der Formel

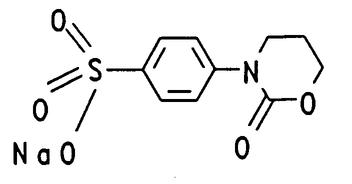

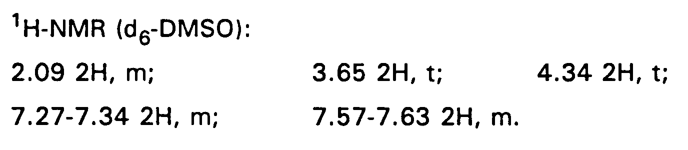

### Stufe 3

90 Teile Mercaptoethanol werden in einer Inertgasatmosphäre mit 2 Teilen Kaliumcarbonat versetzt. Man streut sodann 313 Teile der Verbindung aus der vorangegangenen Stufe 1 und 2 ein und erhitzt sodann auf 190 bis 200°C. Es wird noch eine Weile nachgerührt, bis die Umsetzung beendet ist. Man erhält eine Verbindung der Formel 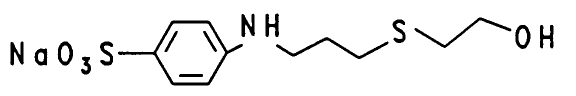 durch Einrühren des erhaltenen Öls in Ethanol, wobei das Produkt als weißer Feststoff ausfällt. Es wird filtriert, mit Ethanol gewaschen und im Vakuum getrocknet.

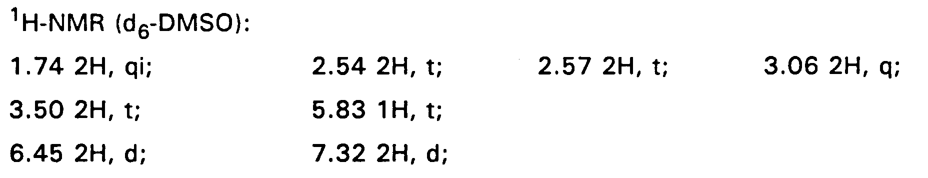

### Stufe 4 und 5 kombiniert:

238 Teile der Verbindung aus Stufe 3 werden in 500 Teile Dimethylacetamid eingetragen, unter Rühren gelöst und mit 1 Teil Na₂WO₄ versetzt. Man gibt dann bei einer Temperatur von 80 bis 90°C 130 Teile Wasserstoffperoxid (35 gew.-%ige wäßrige Lösung) portionsweise hinzu und rührt nach, bis die Umsetzung beendet ist. Man destilliert vorhandenes Wasser im Vakuum ab. Sodann werden langsam bei einer Temperatur von 10 bis 20°C 116 Teile Chlorsulfonsäure zugetropft. Man erhält die Verbindung der Formel 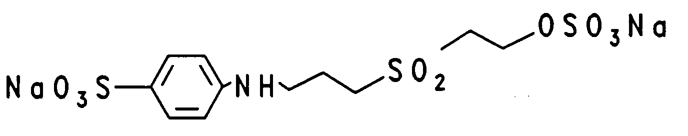 die wie in Beispiel 1 beschrieben, isoliert wird.

### Beispiel 4 Ar = 5-Amino-4-sulfo-phenyl

### Stufe 1 + 2 kombiniert:

Zu 188 Teilen des Natriumsalzes von 2,4-Diamino-benzolsulfonsäure in 1000 Teilen Wasser werden bei einem pH-Wert von 7 innerhalb von 2 bis 3 Stunden 165 Teile Chlorameisensäure-3-chlorpropylester bei 20-25°C zugetropft, wobei der pH-Wert mit 10 gew.-%iger Sodalösung auf 6-7 gehalten wird. Man rührt noch einige Zeit nach, bis die Umsetzung beendet ist und erhöht sodann den pH-Wert auf 11,5 bis 12. Bei einer Temperatur von 70°C wird bei diesem pH-Wert der Ringschluß zu Ende geführt. Der Ansatz wird im Vakuum eingedampft und man erhält die Verbindung 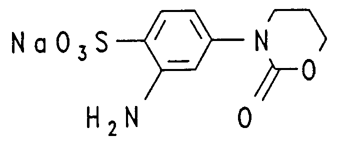 als weißen Feststoff.

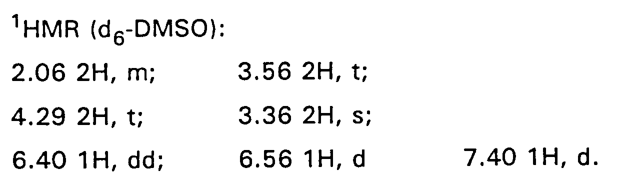

### Stufe 3

240 Teile Mercaptoethanol werden in einer Inertgasatmosphäre mit 0,1 Teilen Kaliumcarbonat versetzt. Man streut sodann 294 Teile der Verbindung aus Stufe 1 + 2 ein und erhitzt sodann auf 160-170°C. Die Umsetzung wird in 2 bis 3 Stunden zu Ende geführt. Man erhält eine neue Thioether-Verbindung der Formel 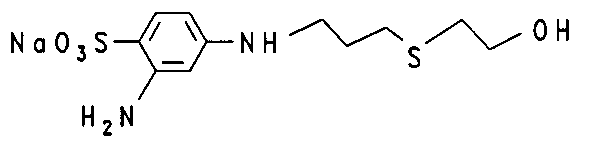 die durch Einrühren des erhaltenen Öls in Ethanol isoliert wird, wobei das Produkt als weißer Feststoff ausfällt. Es wird filtriert, mit mehreren Portionen Ethanol gewaschen und im Vakuum getrocknet.

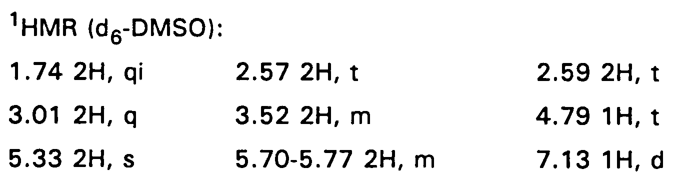

### Stufe 4 + 5 kombiniert:

328 Teile der Verbindung aus Stufe 3 werden in 1000 Teile Wasser eingetragen, unter Rühren gelöst und mit 125 Teilen Acetanhydrid versetzt. Man acyliert bei einem pH-Wert von 3-4 und einer Temperatur von 70-80°C innerhalb 1 Stunde.
Anschließend setzt man 1 Teil Natriumwolframat hinzu. Man gibt sodann bei einer Temperatur von 80-90°C 130 Teile Wasserstoffperoxid portionsweise hinzu und rührt nach, bis die Umsetzung beendet ist. Dabei erhält man die Verbindung der Formel 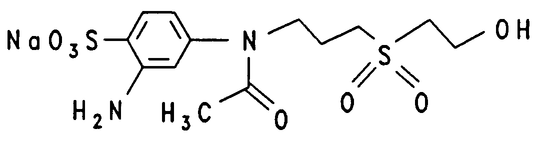 in Mischung mit der entsprechenden an N und O acylierten Verbindung. Es wird nicht isoliert, sondern direkt weiter gearbeitet.
Hierzu werden 150 Teile konzentrierter Schwefelsäure hinzugesetzt und der Ansatz in Inertgasatmosphäre 4 Stunden bei 90-100°C erhitzt, wobei die Acetyl-Schutzgruppe hydrolytisch wieder abspalten wird.
Nach Abkühlen auf 40-50°C destilliert man vorhandenes Wasser und entstandene Essigsäure im Vakuum ab, bis keine flüchtigen Bestandteile mehr übergehen, versetzt den Sumpf mit 500 Teilen N-Methylpyrrolidon und tropft zu der Reaktionslösung langsam bei einer Temperatur von 10-20°C 130 Teile Chlorsulfonsäure hinzu. Anschließend wird noch einige Zeit nachgerührt und durch Einstreuen von festem Soda neutralisiert. Man erhält die Verbindung der Formel 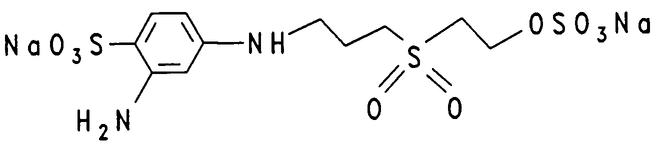 als Natriumsalz, die wie in Beispiel 1 beschrieben als ca. 30%ige Lösung in N-Methylpyrrolidon isoliert wird.

Weitere wertvolle Verbindungen der Formel (1) werden erhalten, wenn man anstelle der in den Beispielen 1 bis 4 genannten Amine in Stufe 1 Amine der Formel Ar-NH₂ einsetzt, wobei der Rest Ar die in der Tabelle genannten Bedeutungen besitzt.

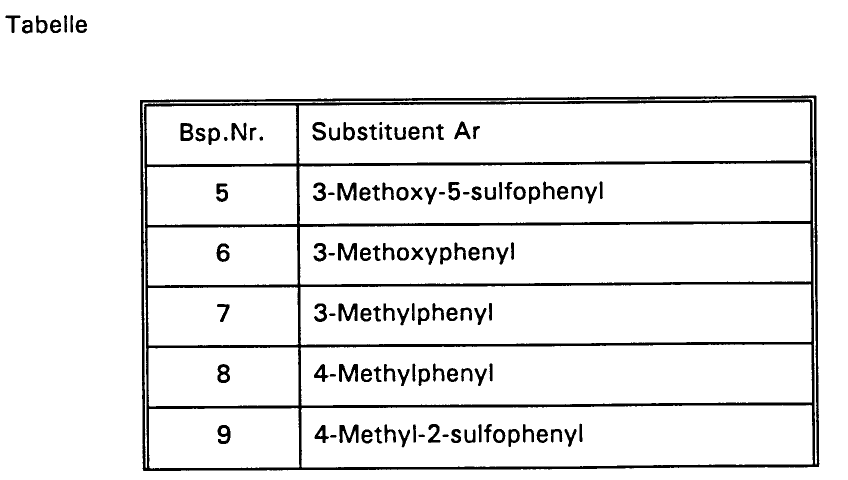

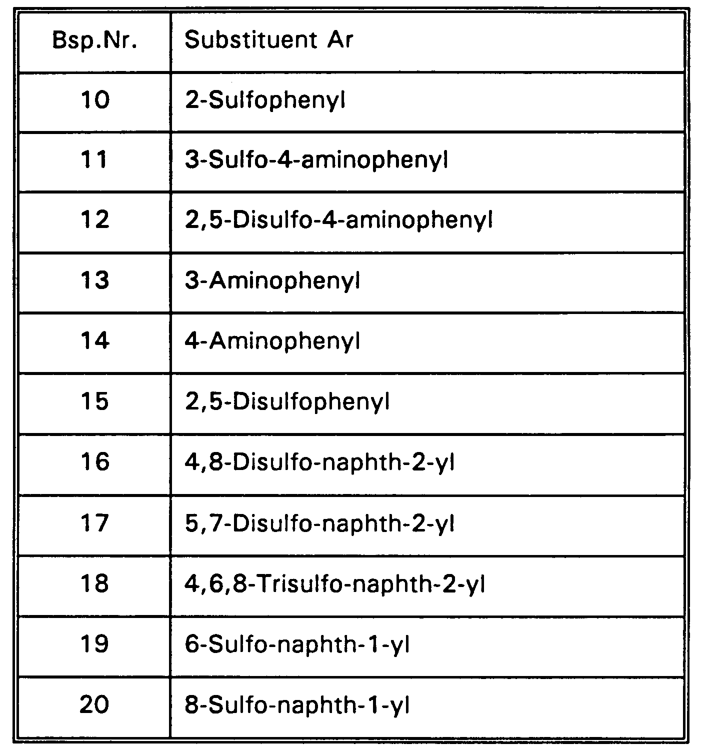

Erklärung der NMR-Kopplungen:
s - Singulett; t - Triplett, q - Quartett; qi - Quintett; m - Multiplett; dd - Doppel-Dublett; dt - Doppel-Triplett.

## Patentansprüche

1. Verfahren zur Herstellung einer Verbindung der Formel (1)
Ar-NH-(CH₂)₃-SO₂-(CH₂)₂-OSO₃M (1)
worin
M für Alkalimetall oder für Wasserstoff steht,
Ar für einen aromatischen Rest der Formel 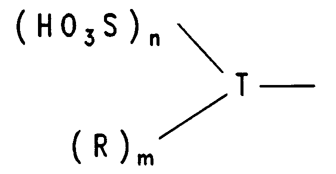
steht, worin
n für eine Zahl von 0 bis 3 steht,
m für eine Zahl von 0 bis 3 steht,
R für C₁-C₄-Alkyl, C₁-C₄-Alkoxy, Amino, C₁-C₄-Alkylamino, Di-(C₁-C₄)-alkylamino, C₆-C₁₀-Arylamino, C₁-C₄-Acylamino, Ureido, Nitro oder Cyano steht, wobei C₁-C₄-Alkyl und C₆-C₁₀-Aryl durch ein bis drei Reste aus der Reihe C₁-C₄-Alkoxy, Halogen, Hydroxy, Carboxy, Sulfo, Sulfato, Nitro, Cyano oder eine Kombination davon substituiert sein können,
und
T für ein aromatisches Brückenglied aus der Benzol- oder Naphthalinreihe
steht,
dadurch gekennzeichnet, daß man eine Verbindung der Formel (3)
Ar-NH-(CH₂)₃-S-(CH₂)₂-OH (3)
zu einer Verbindung der Formel (4) oxidiert
Ar-NH-(CH₂)₃-SO₂-(CH₂)₂-OH (4),
und die Verbindung der Formel (4) mit mindestens einem Mol-Äquivalent Schwefelsäure, Oleum oder Halogensulfonsäure zu einer Verbindung der Formel (1) verestert.

2. Verfahren gemäß Anspruch 1, dadurch gekennzeichnet, daß die Verbindung der Formel (3) durch Umsetzung einer Verbindung der Formel 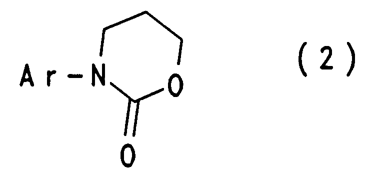 (2) mit 2-Mercaptoethanol bei Temperaturen von 90 bis 270°C in Gegenwart einer katalytischen Menge Base hergestellt wird.

3. Verfahren nach Anspruch 1 oder 2, dadurch gekennzeichnet, daß
M Wasserstoff, Natrium oder Kalium ist,
n für die Zahl 0 oder 1,
m für die Zahl 0 oder 1, vorzugsweise 0,
R für Methyl, Ethyl, Methoxy, Ethoxy, Methylamino oder Amino und
Ar für Phenyl, 2-Sulfophenyl, 3-Sulfophenyl, 4-Sulfophenyl, 2,5-Disulfophenyl, 2-Methylphenyl, 3-Methylphenyl, 4-Methylphenyl, 2-Methoxyphenyl, 3-Methoxyphenyl, 4-Methoxyphenyl, 3-Aminophenyl, 4-Aminophenyl, 3-Sulfo-4-aminophenyl, 4-Sulfo-3-aminophenyl, 3-Methoxy-5-sulfophenyl, 3-Sulfo-4-methylphenyl, 3-Sulfo-4-methoxyphenyl, 2-Sulfo-4-methylphenyl, 4-Methyl-2-sulfophenyl, 2-Sulfo-4-methoxyphenyl, 2,5-Disulfo-4-methylphenyl, 2,5-Disulfo-4-methoxyphenyl, 2,5-Disulfo-4-aminophenyl, 5,7-Disulfo-naphth-2-yl, 4,8-Disulfo-naphth-2-yl, 3,6,8-Trisulfo-naphth-2-yl, 4,6,8-Trisulfo-naphth-2-yl, 8-Sulfo-naphth-1-yl, 6-Sulfo-naphth-1-yl oder 7-Sulfo-naphth-1-yl stehen.

4. Verfahren nach mindestens einem der Ansprüche 1 bis 3, dadurch gekennzeichnet, daß in den Verbindungen der Formeln (1), (2), (3) und (4) Ar die Bedeutung Phenyl, 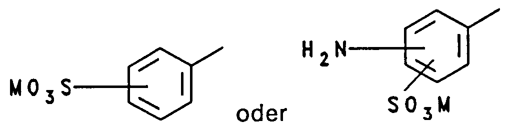 hat und M Wasserstoff oder Alkalimetall, vorzugsweise Na, bedeutet.

5. Verfahren insbesondere nach einem der Ansprüche 1, 2, 3 oder 4 zur Herstellung einer Verbindung der Formel (3)
Ar-NH-(CH₂)₃-S-(CH₂)₂-OH (3)
worin Ar die in Anspruch 1 oder 3 genannte Bedeutung hat, dadurch gekennzeichnet, daß man eine Verbindung der Formel (2) 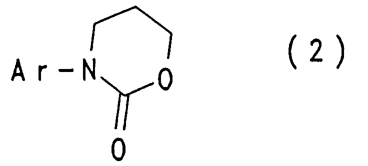 mit 2-Mercaptoethanol im molaren Verhältnis von 1:1 bis 1:3 und in Gegenwart von 1 bis 15 Mol-%, bezogen auf die Verbindung der Formel (2), eines Alkalihydroxids, Erdalkalihydroxids, Alkalicarbonats, Erdalkalicarbonats, Alkaliphosphats oder Alkalialkoholats bei einer Temperatur von 90 bis 270°C umsetzt.

6. Verfahren nach mindestens einem der Ansprüche 1 bis 5, dadurch gekennzeichnet, daß man die Verbindung der Formel (3) mit Wasserstoffperoxid und in Gegenwart einer katalytischen Menge einer Übergangsmetall-Sauerstoff-Verbindung bei einer Temperatur zwischen 70 und 110°C zu einer Verbindung der Formel (4) oxidiert.

7. Verfahren nach mindestens einem der Ansprüche 1 bis 6, dadurch gekennzeichnet, daß man die Verbindung der Formel (4) mit 1 bis 1,5 Molequivalenten, vorzugsweise 1,05 bis 1,2 Molequivalenten, Schwefelsäure, Oleum oder Chlorsulfonsäure bei Temperaturen zwischen 0 und 40°C, vorzugsweise 10 und 30°C, zu einer Verbindung der Formel (1) verestert.

8. Verbindung der Formel (2a) 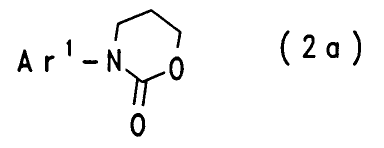 worin
Ar¹ 2-Sulfophenyl, 3-Sulfophenyl, 4-Sulfophenyl, 2,5-Disulfophenyl, 3-Sulfo-4-aminophenyl, 4-Sulfo-3-aminophenyl, 3-Methoxy-5-sulfophenyl, 3-Sulfo-4-methylphenyl, 3-Sulfo-4-methoxyphenyl, 2-Sulfo-4-methylphenyl, 2,5-Disulfo-4-methylphenyl, 2,5-Disulfo-4-methoxyphenyl, 2,5-Disulfo-4-aminophenyl, 5,7-Disulfo-naphth-2-yl, 4,8-Disulfo-naphth-2-yl, 3,6,8-Trisulfo-naphth-2-yl, 4,6,8-Trisulfo-naphth-2-yl, 8-Sulfo-naphth-1-yl, 6-Sulfo-naphth-1-yl oder 7-Sulfo-naphth-1-yl und
M Alkalimetall oder Wasserstoff bedeutet.

9. Verbindung der Formel (3)
Ar-NH-(CH₂)₃-S-(CH₂)₂-OH (3)
worin
Ar die Bedeutung Phenyl, 2-Sulfophenyl, 3-Sulfophenyl, 4-Sulfophenyl, 2,5-Disulfophenyl, 2-Methylphenyl, 3-Methylphenyl, 4-Methylphenyl, 2-Methoxyphenyl, 3-Methoxyphenyl, 4-Methoxyphenyl, 3-Aminophenyl, 4-Aminophenyl, 3-Sulfo-4-aminophenyl, 4-Sulfo-3-aminophenyl, 3-Methoxy-5-sulfophenyl, 3-Sulfo-4-methylphenyl, 3-Sulfo-4-methoxyphenyl, 2-Sulfo-4-methylphenyl, 4-Methyl-2-sulfophenyl, 2-Sulfo-4-methoxyphenyl, 2,5-Disulfo-4-methylphenyl, 3,5-Disulfo-4-methoxyphenyl, 2,5-Disulfo-4-aminophenyl, 5,7-Disulfo-naphth-2-yl, 4,8-Disulfo-naphth-2-yl, 3,6,8-Trisulfo-naphth-2-yl, 4,6,8-Trisulfo-naphth-2-yl, 8-Sulfo-naphth-1-yl, 6-Sulfo-naphth-1-yl oder 7-Sulfo-naphth-1-yl hat und M Wasserstoff oder Alkalimetall bedeutet.

## Claims

1. A process for the preparation of a compound of the formula (1)
Ar-NH-(CH₂)₃-SO₂-(CH₂)₂-OSO₃M (1)
in which
M is an alkali metal or hydrogen and
Ar is an aromatic radical of the formula 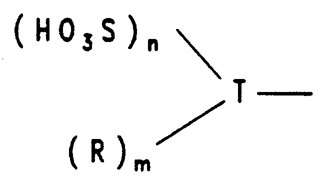
in which
n is a number from 0 to 3,
m is a number from 0 to 3,
R is C₁-C₄-alkyl, C₁-C₄-alkoxy, amino, C₁-C₄-alkylamino, di-(C₁-C₄)-alkylamino, C₆-C₁₀-arylamino, C₁-C₄-acylamino, ureido, nitro or cyano, where C₁-C₄-alkyl and C₆-C₁₀-aryl can be substituted by one to three radicals from the group consisting of C₁-C₄-alkoxy, halogen, hydroxyl, carboxyl, sulfo, sulfato, nitro, cyano or a combination thereof,
and
T is an aromatic bridge member from the benzene or naphthalene series,
which comprises oxidizing a compound of the formula (3)
Ar-NH-(CH₂)₃-S-(CH₂)₂-OH (3)
to give a compound of the formula (4)
Ar-NH-(CH₂)₃-SO₂-(CH₂)₂-OH (4),
and esterifying the compound of the formula (4) with at least one molar equivalent of sulfuric acid, oleum or halosulfonic acid to give a compound of the formula (1).

2. The process as claimed in claim 1, wherein the compound of the formula (3) is prepared by reaction of a compound of the formula (2) 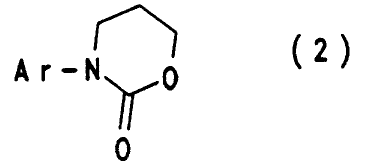 with 2-mercaptoethanol at temperatures from 90 to 270°C in the presence of a catalytic amount of a base.

3. The process as claimed in claim 1 or 2, wherein
M is hydrogen, sodium or potassium,
n is the number 0 or 1,
m is the number 0 or 1, preferably 0,
R is methyl, ethyl, methoxy, ethoxy, methylamino or amino and
Ar is phenyl, 2-sulfophenyl, 3-sulfophenyl, 4-sulfophenyl, 2,5-disulfophenyl, 2-methylphenyl, 3-methylphenyl, 4-methylphenyl, 2-methoxyphenyl, 3-methoxyphenyl, 4-methoxyphenyl, 3-aminophenyl, 4-aminophenyl, 3-sulfo-4-aminophenyl, 4-sulfo-3-aminophenyl, 3-methoxy-5-sulfophenyl, 3-sulfo-4-methylphenyl, 3-sulfo-4-methoxyphenyl, 2-sulfo-4-methylphenyl, 4-methyl-2-sulfophenyl, 2-sulfo-4-methoxyphenyl, 2,5-disulfo-4-methylphenyl, 2,5-disulfo-4-methoxyphenyl, 2,5-disulfo-4-aminophenyl, 5,7-disulfo-naphth-2-yl, 4,8-disulfo-naphth-2-yl, 3,6,8-trisulfo-naphth-2-yl, 4,6,8-trisulfo-naphth-2-yl, 8-sulfo-naphth-1-yl, 6-sulfo-naphth-1-yl or 7-sulfo-naphth-1-yl.

4. The process as claimed in at least one of claims 1 to 3, wherein, in the compounds of the formulae (1), (2), (3) and (4),
Ar is phenyl, 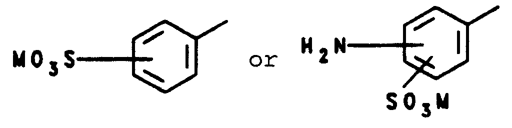 and
M is hydrogen or an alkali metal, preferably Na.

5. The process in particular as claimed in one of claims 1, 2, 3 or 4 for the preparation of a compound of the formula (3)
Ar-NH-(CH₂)₃-S-(CH₂)₂-OH (3)
in which Ar has the meaning given in claim 1 or 3, wherein a compound of the formula (2) 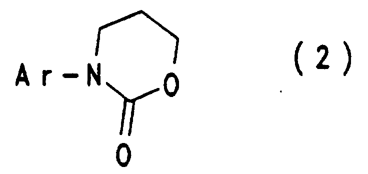 is reacted with 2-mercaptoethanol in a molar ratio of 1:1 to 1:3 and in the presence of 1 to 15 mol%, based on the compound of the formula (2), of an alkali metal hydroxide, alkaline earth metal hydroxide, alkali metal carbonate, alkaline earth metal carbonate, alkali metal phosphate or alkali metal alcoholate at a temperature from 90 to 270°C.

6. The process as claimed in at least one of claims 1 to 5, wherein the compound of the formula (3) is oxidized with hydrogen peroxide and in the presence of a catalytic amount of a transition metal-oxygen compound at a temperature between 70 and 110°C to give a compound of the formula (4).

7. The process as claimed in at least one of claims 1 to 6, wherein the compound of the formula (4) is esterified with 1 to 1.5 molar equivalents, preferably 1.05 to 1.2 molar equivalents, of sulfuric acid, oleum or chlorosulfonic acid at temperatures between 0 and 40°C, preferably 10 and 30°C, to give a compound of the formula (1).

8. A compound of the formula (2a) 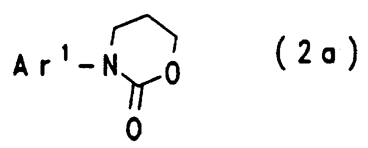 in which
Ar¹ is 2-sulfophenyl, 3-sulfophenyl, 4-sulfophenyl, 2,5-disulfophenyl, 3-sulfo-4-aminophenyl, 4-sulfo-3-aminophenyl, 3-methoxy-5-sulfophenyl, 3-sulfo-4-methylphenyl, 3-sulfo-4-methoxyphenyl, 2-sulfo-4-methylphenyl, 2,5-disulfo-4-methylphenyl, 2,5-disulfo-4-methoxyphenyl, 2,5-disulfo-4-aminophenyl, 5,7-disulfo-naphth-2-yl, 4,8-disulfo-naphth-2-yl, 3,6,8-trisulfo-naphth-2-yl, 4,6,8-trisulfo-naphth-2-yl, 8-sulfo-naphth-1-yl, 6-sulfo-naphth-1-yl or 7-sulfo-naphth-1-yl and
M is an alkali metal or hydrogen.

9. A compound of the formula (3)
Ar-NH-(CH₂)₃-S-(CH₂)₂-OH (3)
in which
Ar is phenyl, 2-sulfophenyl, 3-sulfophenyl, 4-sulfophenyl, 2,5-disulfophenyl, 2-methylphenyl, 3-methylphenyl, 4-methylphenyl, 2-methoxyphenyl, 3-methoxyphenyl, 4-methoxyphenyl, 3-aminophenyl, 4-aminophenyl, 3-sulfo-4-aminophenyl, 4-sulfo-3-aminophenyl, 3-methoxy-5-sulfophenyl, 3-sulfo-4-methylphenyl, 3-sulfo-4-methoxyphenyl, 2-sulfo-4-methylphenyl, 4-methyl-2-sulfophenyl, 2-sulfo-4-methoxyphenyl, 2,5-disulfo-4-methylphenyl, 3,5-disulfo4-methoxyphenyl, 2,5-disulfo-4-aminophenyl, 5,7-disulfo-naphth-2-yl, 4,8-disulfo-naphth-2-yl, 3,6,8-trisulfo-naphth-2-yl, 4,6,8-trisulfo-naphth-2-yl, 8-sulfo-naphth-1-yl, 6-sulfo-naphth-1-yl or 7-sulfo-naphth-1-yl and M is hydrogen or an alkali metal.

## Revendications

1. Procédé de préparation d'un composé de formule (1) :
Ar-NH-(CH₂)₃-SO₂-(CH₂)₂-OSO₃M (1)
dans laquelle :
M représente un métal alcalin ou un hydrogène;
Ar représente un radical aromatique de formule : 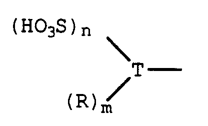 dans laquelle :
n représente un nombre de 0 à 3;
m représente un nombre de 0 à 3;
R représente un alcoyle en C₁-C₄, un alcoxy en C₁-C₄, un amino, un (alcoyl en C₁-C₄)amino, un di(alcoyl en C₁-C₄)amino, un (aryl en C₆-C₁₀)amino, un (acyl en C₁-C₄)amino, un uréido, un nitro ou un cyano, l'alcoyle en C₁-C₄ et l'aryle en C₆-C₁₀ pouvant être substitués par un à trois radicaux de la série d'un alcoxy en C₁-C₄, d'un halogène, d'un hydroxy, d'un carboxy, d'un sulfo, d'un sulfato, d'un nitro, d'un cyano ou d'une combinaison de ceux-ci, et
T représente un élément aromatique en pont de la série du benzène ou du naphtalène,
caractérisé en ce que l'on oxyde un composé de formule (3) :
Ar-NH-(CH₂)₃-S-(CH₂)₂-OH (3)
en un composé de formule (4) :
Ar-NH-(CH₂)₃-SO₂-(CH₂)₂-OH (4)
et dans lequel le composé de formule (4) est estérifié en un composé de formule (1) avec au moins un équivalent molaire d'acide sulfurique, d'oléum ou d'acide halogénosulfonique.

2. Procédé suivant la revendication 1, caractérisé en ce que le composé de formule (3) est préparé par réaction d'un composé de formule (2) : 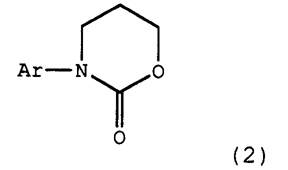 avec du 2-mercaptoéthanol, à des températures allant de 90 à 270°C, en présence d'une quantité catalytique de base.

3. Procédé suivant la revendication 1 ou 2, caractérisé en ce que :
M est l'hydrogène, le sodium ou le potassium;
n représente le nombre 0 ou 1;
m représente le nombre 0 ou 1, de préférence 0;
R représente un méthyle, un éthyle, un méthoxy, un éthoxy, un méthylamino ou un amino, et
Ar représente un phényle, un 2-sulfophényle, un 3-sulfophényle, un 4-sulfophényle, un 2,5-disulfophényle, un 2-méthylphényle, un 3-méthylphényle, un 4-méthylphényle, un 2-méthoxyphényle, un 3-méthoxyphényle, un 4-méthoxyphényle, un 3-aminophényle, un 4-aminophényle, un 3-sulfo-4-aminophényle, un 4-sulfo-3-aminophényle, un 3-méthoxy-5-sulfophényle, un 3-sulfo-4-méthylphényle, un 3-sulfo-4-méthoxyphényle, un 2-sulfo-4-méthylphényle, un 4-méthyl-2-sulfophényle, un 2-sulfo-4-méthoxyphényle, un 2,5-disulfo-4-méthylphényle, un 2,5-disulfo-4-méthoxyphényle, un 2,5-disulfo-4-aminophényle, un 5,7-disulfonapht-2-yle, un 4,8-disulfonapht-2-yle, un 3,6,8-trisulfonapht-2-yle, un 4,6,8-trisulfonapht-2-yle, un 8-sulfonapht-1-yle, un 6-sulfonapht-1-yle ou un 7-sulfonapht-1-yle.

4. Procédé suivant au moins l'une des revendications 1 à 3, caractérisé en ce que, dans les composés de formules (1), (2), (3) et (4), Ar signifie phényle, 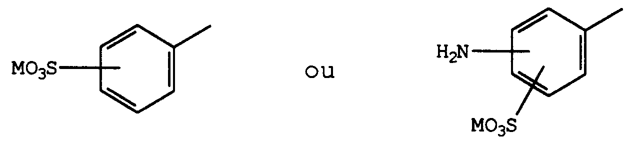 et M signifie l'hydrogène ou un métal alcalin, de préférence Na.

5. Procédé en particulier suivant l'une des revendications 1, 2, 3 ou 4, pour la préparation d'un composé de formule (3) :
Ar-NH-(CH₂)₃-S-(CH₂)₂-OH (3)
dans laquelle Ar a la signification donnée dans la revendication 1 ou 3, caractérisé en ce que l'on fait réagir un composé de formule (2) : 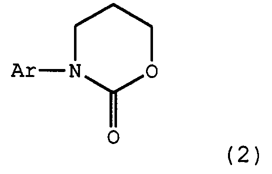 avec du 2-mercaptoéthanol dans le rapport molaire allant de 1:1 à 1:3 et en présence de 1 à 15% en mole, sur la base du composé de formule (2), d'un hydroxyde alcalin, d'un hydroxyde alcalino-terreux, d'un carbonate alcalin, d'un carbonate alcalino-terreux, d'un phosphate alcalin ou d'un alcoolate alcalin, à une température de 90 à 270°C.

6. Procédé suivant au moins l'une des revendications 1 à 5, caractérisé en ce que l'on oxyde le composé de formule (3) en un composé de formule (4) avec du peroxyde d'hydrogène et en présence d'une quantité catalytique d'un composé métal de transitionoxygène à une température comprise entre 70 et 110°C.

7. Procédé suivant au moins l'une des revendications 1 à 6, caractérisé en ce que l'on estérifie le composé de formule (4) en un composé de formule (1) avec 1 à 1,5 équivalents molaires, de préférence 1,05 à 1,2 équivalents molaires, d'acide sulfurique, d'oléum ou d'acide chlorosulfonique, à des températures comprises entre 0 et 40°C, de préférence entre 10 et 30°C.

8. Composé de formule (2a) : 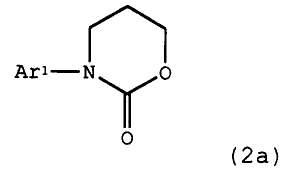 dans laquelle :
Ar¹ représente un 2-sulfophényle, un 3-sulfophényle, un 4-sulfophényle, un 2,5-disulfophényle, un 3-sulfo-4-aminophényle, un 4-sulfo-3-aminophényle, un 3-méthoxy-5-sulfophényle, un 3-sulfo-4-méthylphényle, un 3-sulfo-4-méthoxyphényle, un 2-sulfo-4-méthylphényle, un 2,5-disulfo-4-méthylphényle, un 2,5-disulfo-4-méthoxyphényle, un 2,5-disulfo-4-aminophényle, un 5,7-disulfonapht-2-yle, un 4,8-disulfonapht-2-yle, un 3,6,8-trisulfonapht-2-yle, un 4,6,8-trisulfonapht-2-yle, un 8-sulfonapht-1-yle, un 6-sulfonapht-1-yle ou un 7-sulfonapht-1-yle, et
M représente un métal alcalin ou l'hydrogène.

9. Composé de formule (3) :
Ar-NH-(CH₂)₃-S-(CH₂)₂-OH (3)
dans laquelle :
Ar signifie un phényle, un 2-sulfophényle, un 3-sulfophényle, un 4-sulfophényle, un 2,5-disulfophényle, un 2-méthylphényle, un 3-méthylphényle, un 4-méthylphényle, un 2-méthoxyphényle, un 3-méthoxyphényle, un 4-méthoxyphényle, un 3-aminophényle, un 4-aminophényle, un 3-sulfo-4-aminophényle, un 4-sulfo-3-aminophényle, un 3-méthoxy-5-sulfophényle, un 3-sulfo-4-méthylphényle, un 3-sulfo-4-méthoxyphényle, un 2-sulfo-4-méthylphényle, un 4-méthyl-2-sulfophényle, un 2-sulfo-4-méthoxyphényle, un 2,5-disulfo-4-méthylphényle, un 3,5-disulfo-4-méthoxyphényle, un 2,5-disulfo-4-aminophényle, un 5,7-disulfonapht-2-yle, un 4,8-disulfonapht-2-yle, un 3,6,8-trisulfonapht-2-yle, un 4,6,8-trisulfonapht-2-yle, un 8-sulfonapht-1-yle, un 6-sulfonapht-1-yle ou un 7-sulfonapht-1-yle, et M signifie un hydrogène ou un métal alcalin.
